# EUROPEAN PATENT APPLICATION

(11) **EP 4 494 634 A1**
(43) Date of publication of application: **22.01.2025**
(21) Application number: 23795162.9
(22) Date of filing: 19.04.2023
(51) Int. Cl.: A61K 9/08, A61K 31/135, A61K 47/28, A61K 47/38, A61P 25/24

(54) **ESKETAMINE LIQUID PREPARATION AND USE THEREOF**

(30) Priority: 26.04.2022 CN 202210450509
(71) Applicant: Yichang Humanwell Pharmaceutical Co., Ltd., Yichang, Hubei 443005 (CN)
(72) Inventor: WU, Jihong, Yichang, Hubei 443005 (CN); HUANG, Zhiyong, Yichang, Hubei 443005 (CN); XIONG, Jin, Yichang, Hubei 443005 (CN); WU, Youbin, Yichang, Hubei 443005 (CN); LIU, Peng, Yichang, Hubei 443005 (CN); LI, Ke, Yichang, Hubei 443005 (CN); LI, Xiaomei, Yichang, Hubei 443005 (CN); LV, Jinliang, Yichang, Hubei 443005 (CN); LI, Lie, Yichang, Hubei 443005 (CN); QU, Qin, Yichang, Hubei 443005 (CN); CHEN, Yi, Yichang, Hubei 443005 (CN)
(74) Representative: Meissner Bolte Nürnberg
(86) International application number: PCT/CN2023/089327
(87) International publication number: WO 2023/207729

(57) **Abstract**

An esketamine liquid preparation. The esketamine liquid preparation is an aqueous solution agent, comprising esketamine or a pharmaceutically acceptable salt thereof, cholic acid or a derivative thereof, and water, with a pH value of 2.5-5.7. The esketamine liquid preparation is administrated via oral mucosa, and the preparation is high in stability, fast in absorption, high in bioavailability, simple in administration mode, and good in compliance. The esketamine liquid preparation can be used for preventing, relieving, or treating major depression, unipolar depression, treatment-refractory depression, intractable depression, agitated depression, and bipolar depression.

## Description

This application claims priority to a Chinese patent application filed in the Chinese Patent Office on April 26, 2022, with application number 202210450509.0 and the invention title "Esketamine Liquid Formulation and Use thereof", the contents of which should be understood to be incorporated herein by reference.

### Technical Field

The present application relates to, but is not limited to, the technical field of pharmaceutical formulations, and in particular to esketamine liquid formulations and their use as antidepressants.

### Background

Depression is an episode form of manic depression, characterized by typical symptoms of low emotion, delayed thinking, and reduced and delayed speech movements. Depression seriously troubles patients' life and work, and brings a heavy burden to families and society. About 15% of patients with depression die of suicide. A joint study by the World Health Organization, the World Bank and Harvard University shows that depression has become the second largest disease in China's disease burden.

In recent years, ketamine has attracted much attention as a psychiatric drug to treat depression, and a large number of reports have proved its antidepressant effect. As a fast-acting antidepressant, ketamine may take effect through the following mechanism: increasing the release of monoamines or inhibiting presynaptic reuptake of monoamines, thus increasing the level of monoamine compounds in the brain to achieve antidepressant effects.

Ketamine is an N-methyl-D-aspartate (NMDA) receptor antagonist. Currently, the ketamine for clinical use is used mainly as racemate, which includes two enantiomers, namely D-ketamine and L-ketamine. Studies have shown that the affinity of D-ketamine, namely Esketamine, for NMDA, opioid receptors, and M-choline receptors is about 2 to 4 times higher than that of L-ketamine, while the inhibitory effect on 5-HT is only 50% of L-ketamine. In addition, the side effects such as hallucinations and dreams appeared in the application of ketamine are mainly caused by the levorotatory isomer. Therefore, esketamine has better curative effect and fewer side effects such as hallucinogenic action and addiction.

An antidepressant of new mechanism developed by Johnson & Johnson in the United States is a nasal spray with esketamine hydrochloride as the main drug. This product has quick-acting and long-lasting powerful curative effect for patients with refractory depression and suicidal tendencies, and was approved for its first marketing in the United States in March 2019. In addition, the product was approved as Breakthrough Therapy Designation (BTD) for the treatment of refractory depression and Breakthrough Therapy Designation for the treatment of major depression accompanied with imminent suicide risk granted by the FDA in 2013 and 2016, respectively.

Although esketamine has achieved a good antidepressant effect in the form of nasal administration, nasal administration cannot meet the medicinal needs of patients with inflammation of the nasal cavity or poor tolerance of the nasal mucosa. In addition, for the marketed nasal spray formulations of esketamine hydrochloride, the volume of nasal administration is small, and in order to achieve the therapeutic dose, the concentration of esketamine hydrochloride in the formulations is high, which is close to the water saturation of esketamine. This high concentration of esketamine hydrochloride solution increases the risk of ketamine abuse to some extent. Furthermore, through pharmacokinetic studies, it was found that when esketamine was administered by nasal spray, the drug was absorbed quickly, however, the drug absorption in the body was not good, and the bioavailability was not high. Therefore, in order to further expand the scope of clinical application of ketamine in anti-depression, meet the needs of more patients with depression, reduce the treatment cost, and reduce the burden of patients' families, it is urgent to consider other effective routes of administration.

### Summary

The present application provides an esketamine liquid formulation for oral mucosa administration. The formulation of the present application has high stability, rapid absorption, high bioavailability, simple application mode and good compliance.

In one aspect, the present application provides an esketamine liquid formulation for oral mucosa administration, comprising esketamine or a pharmaceutically acceptable salt thereof, cholic acid or a derivative thereof, and water.

In another aspect, the present application provides a use of the aforementioned esketamine liquid formulation in the manufacture of a medicament for preventing, alleviating or treating depression, the medicament can be used for treating major depression, unipolar depression, refractory depression, intractable depression, anxiety depression, or bipolar depression.

In yet another aspect, the present application provides a method for preventing, alleviating or treating depression in a patient using the aforementioned esketamine liquid formulation, comprising administering to the patient a therapeutically effective amount of the aforementioned esketamine liquid formulation.

In yet another aspect, the present application provides the aforementioned esketamine liquid formulation for use in preventing, alleviating or treating depression in a patient, the depression is major depression, unipolar depression, refractory depression, intractable depression, anxiety depression, or bipolar depression.

In yet another aspect, the present application provides a method for preparing the aforementioned esketamine liquid formulation.

### Brief Description of Drawings

The accompanying drawings are used to provide an understanding of the technical solutions of the present application, and constitute a part of the specification. They are used to explain the technical solutions of the present application together with the embodiments of the present application, and do not constitute a limitation to the technical solutions of the present application.
FIG. 1 is a diagram of the relationship between administration routes and pharmacokinetics of different esketamine formulations proposed in the present application.
FIG. 2 is a diagram of the relationship between different administration routes and AUC of different esketamine formulations proposed in the present application.

### Detailed Description

A first aspect of the present application provides an esketamine liquid formulation for oral mucosa administration, comprising esketamine or a pharmaceutically acceptable salt thereof, cholic acid or a derivative thereof, and water.

Unless otherwise specified, esketamine as used herein, also referred to as S-ketamine, Esketamine, (S)-2-(2-chlorophenyl)-2-(methylamino) cyclohexanone hydrochloride, and the like, refers to the (S)-enantiomer of ketamine.

In the liquid formulation of the present invention, whether esketamine or a pharmaceutically acceptable salt of esketamine is used, when calculating the content of active substance, the content of esketamine is calculated.

In one embodiment of the esketamine liquid formulation of the present application, the content of esketamine in the liquid formulation is 0.5% w/v - 8.5% w/v.

Since the esketamine liquid formulation described in the present application has high bioavailability, the liquid formulation can be prepared as a product with a lower substance concentration, which cannot only meet the preventive or therapeutic needs of clinical patients, but also avoid the risk of abuse caused by high concentration administration. At the same time, esketamine liquid formulation has good compatibility with saliva, which reduces the irritation to mucous membrane and reduces the side effects of drugs.

In one embodiment, the concentration of esketamine in the liquid formulation is 0.7% w/v - 8.4% w/v. In another embodiment, the concentration of esketamine in the liquid formulation is 1.4% w/v - 6.3% w/v. In yet another embodiment, the concentration of esketamine in the liquid formulation is 1.4% w/v - 5.6% w/v. In still one embodiment, the concentration of esketamine in the liquid formulation is 0.7% w/v, 0.8% w/v, 0.9% w/v, 1.0% w/v, 1.1% w/v, 1.2% w/v, 1.3% w/v, 1.4% w/v, 1.5% w/v, 1.6% w/v, 1.7% w/v, 1.8% w/v, 1.9% w/v, 2.0% w/v, 2.1% w/v, 2.2% w/v, 2.3% w/v, 2.4% w/v, 2.5% w/v, 2.6% w/v, 2.7% w/v, 2.8% w/v, 2.9% w/v, 3.0% w/v, 3.1% w/v, 3.2% w/v, 3.3% w/v, 3.4% w/v, 3.5% w/v, 3.6% w/v, 3.7% w/v, 3.8% w/v, 3.9% w/v, 4.0% w/v, 4.1% w/v, 4.2% w/v, 4.3% w/v, 4.4% w/v, 4.5% w/v, 4.6% w/v, 4.7% w/v, 4.8% w/v, 4.9% w/v, 5.0% w/v, 5.1% w/v, 5.2% w/v, 5.3% w/v, 5.4% w/v, 5.5% w/v, 5.6% w/v, 5.7% w/v, 5.8% w/v, 5.9% w/v, 6.0% w/v, 6.1% w/v, 6.2% w/v, 6.3% w/v, 6.4% w/v, 6.5% w/v, 6.6% w/v, 6.7% w/v, 6.8% w/v, 6.9% w/v, 7.0% w/v, 7.1% w/v, 7.2% w/v, 7.3% w/v, 7.4% w/v, 7.5% w/v, 7.6% w/v, 7.7% w/v, 7.8% w/v, 7.9% w/v, 8.0% w/v, 8.1% w/v, 8.2% w/v, 8.3% w/v, 8.4% w/v.

In an embodiment, acids that can form pharmaceutically acceptable salts with esketamine include hydrochloric acid, sulfuric acid, phosphoric acid, maleic acid, acetic acid, adipic acid, alginic acid, citric acid, aspartic acid, benzoic acid, benzenesulfonic acid, bisulfuric acid, butyric acid, camphoric acid, camphorsulfonic acid, glucaric acid, fumaric acid, glycerophosphoric acid, stearic acid, heptanoic acid, hexanoic acid, hydrobromic acid (i.e., HBr), hydroiodic acid (i.e., HI), lactic acid, methanesulfonic acid, nicotinic acid, oxalic acid, dihydroxynaphthoic acid, pectic acid, persulfuric acid, 3-phenylpropionic acid, picric acid, pivalic acid, propionic acid, succinic acid, tartaric acid, thiocyanic acid, glutamic acid, p-toluenesulfonic acid, undecanoic acid, and mandelic acid.

In an embodiment, pharmaceutically acceptable salts of esketamine include hydrochloride, sulfate, phosphate, maleate, acetate, adipate, alginate, citrate, aspartate, benzoate, benzenesulfonate, bisulfate, butyrate, camphorate, camphorsulfonate, glucarate, fumarate, glycerophosphate, stearate, heptanoate, hexanoate, salt of hydrobromic acid (i.e., HBr), salt of hydroiodic acid (i.e., HI), lactate, mesylate, nicotinate, oxalate, dihydroxynaphthate, pectate, persulfate, 3-phenylpropionate, picrate, pivalate, propionate, succinate, tartrate, thiocyanate, glutamate, bicarbonate, p-toluenesulfonate, undecanoate, and mandelate.

In one embodiment, the concentration of cholic acid or derivative thereof in the liquid formulation is 0.001% w/v - 0.015% w/v. In another embodiment, the concentration of cholic acid or derivative thereof in the liquid formulation is 0.001% w/v - 0.013% w/v. In yet another embodiment, the concentration of cholic acid or derivative thereof in the liquid formulation is 0.001% w/v - 0.012% w/v. In still one embodiment, the concentration of cholic acid or derivative thereof in the liquid formulation is 0.001% w/v, 0.002% w/v, 0.003% w/v, 0.004% w/v, 0.005% w/v, 0.006% w/v, 0.007% w/v, 0.008% w/v, 0.009% w/v or 0.010% w/v.

In one embodiment, the cholic acid or derivative thereof is one of sodium taurate, sodium taurodeoxycholate, sodium deoxycholate, sodium cholate, sodium glycinodeoxycholate, or a combination thereof.

In one embodiment, the liquid formulation has a pH of 2.5 - 5.7. In another embodiment, the liquid formulation has a pH in the range of 3.0 - 5.7. In yet another embodiment, the esketamine liquid formulation has a pH in the range of 4.0- 5.7. In yet another embodiment, the liquid formulation has a pH in the range of 5.0 - 5.5. In still one embodiment, the liquid formulation has a pH of 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, or 5.7.

In one embodiment, the liquid formulation comprises esketamine or a pharmaceutically acceptable salt thereof with a concentration of 0.5% w/v - 8.5% w/v, cholic acid or a derivative thereof with a concentration of 0.001% w/v - 0.015% w/v, and water, and the liquid formulation has a pH in the range of 2.5 to 5.7.

In some embodiments of the present application, the esketamine liquid formulation may further comprise a tackifier.

In one embodiment, the tackifier is xanthan gum, sodium carboxymethyl cellulose, hydroxypropyl methyl cellulose, methyl cellulose, ethyl cellulose, hydroxyethyl cellulose, polyvinyl alcohol, carbomer, or polyvinylpyrrolidone, or a combination thereof.

In a certain embodiment, the tackifier sodium carboxymethyl cellulose is selected from one or more of sodium carboxymethyl cellulose 800, sodium carboxymethyl cellulose 4000, sodium carboxymethyl cellulose 8000, sodium carboxymethyl cellulose 12000.

In one embodiment, the combination of tackifiers comprises, without limitation, sodium carboxymethyl cellulose 4000 and sodium carboxymethyl cellulose 12000, sodium carboxymethyl cellulose 800 and sodium carboxymethyl cellulose 12000, sodium carboxymethyl cellulose 4000 and xanthan gum, sodium carboxymethyl cellulose 4000 and hydroxypropyl methyl cellulose, sodium carboxymethyl cellulose 4000 and xanthan gum composition, sodium carboxymethyl cellulose 4000 and hydroxypropyl methyl cellulose composition, and the ratio (w/w) of the combination of the two tackifiers is in the range of 1: 5 to 5: 1.

In another embodiment, the concentration of the tackifier is 0.01% w/v - 3.5% w/v. In another embodiment, the concentration of the tackifier is 0.05-3.0% w/v. In yet another embodiment, the concentration of the tackifier is 0.05-2.0% w/v. In still one embodiment, the concentration of the tackifier is 0.01 % w/v, 0.05 % w/v, 0.1 % w/v, 0.15 % w/v, 0.2 % w/v, 0.25 % w/v, 0.30 % w/v, 0.35 % w/v, 0.40 % w/v, 0.45 % w/v, 0.50 % w/v, 0.55 % w/v, 0.60 % w/v, 0.65 % w/v, 0.70 % w/v, 0.75 % w/v, 0.80 % w/v, 0.85 % w/v, 0.90 % w/v, 0.95 % w/v, or 1.00 % w/v.

In another embodiment, the esketamine liquid formulation of further comprises one or more of the following auxiliary materials: a buffer, a flavoring agent, an antioxidant, an osmotic pressure regulator, and a preservative. In another embodiment, the esketamine liquid formulation further comprises one or more of the following auxiliary materials: a buffer, a flavoring agent, an antioxidant, an osmotic pressure regulator, a preservative, and a permeation enhancer.

In an embodiment of the esketamine liquid formulation described in the present application, the buffer may be citric acid, sodium dihydrogen phosphate, disodium hydrogen phosphate, acetic acid, boric acid, sodium borate, succinic acid, tartaric acid, lactic acid, fumaric acid, trisodium phosphate (trisodium phosphate dodecahydrate or TSP), sodium benzoate, benzoic acid, sodium hydroxide, potassium hydroxide, alkali metal carbonate, sodium carbonate, imidazole, pyrophosphate, sodium gluconate, sodium lactate, phosphoric acid, borate, bicarbonate, Tris-HCl, citrate, or a combination thereof.

The buffer described in the present application can maintain the pH of the liquid formulation within a stable range, which is beneficial to improve the stability of the liquid formulation. The concentration of the buffer is from 0.1% to 0.3% w/v. In an embodiment, the concentration of the buffer is from 0.1% to 0.2% w/v. In another embodiment, the concentration of the buffer is from 0.1% to 0.15% w/v. In another embodiment, the concentration of the buffer is 0.1% w/v, 0.11% w/v, 0.12% w/v, 0.13% w/v, 0.14% w/v, 0.15% w/v, 0.16% w/v, 0.17% w/v, 0.18% w/v, 0.19% w/v, 0.2% w/v, 0.21% w/v, 0.22% w/v, 0.23% w/v, 0.24% w/v, 0.25% w/v, 0.26% w/v, 0.27% w/v, 0.28% w/v, 0.29% w/v, or 0.3% w/v.

In another embodiment, the present application provides an esketamine liquid formulation, which further comprises a flavoring agent. The flavoring agent can mask the off-flavor that a pharmaceutical composition may have, and thus improve its palatability, which is beneficial to improving patient compliance. In an embodiment, the flavoring agent of the liquid formulation is sodium saccharin, fructose, sucralose, steviosin, menthol, maltitol, xylitol, aspartame, sodium cyclamate, saccharin, neohesperidin, thaumatin, stevia, or acesulfame, or a combination thereof.

In an embodiment, the present application provides an esketamine liquid formulation, in which the concentration of the flavoring agent is from 0.01% to 0.05% w/v. In another embodiment, the concentration of the flavoring agent is from 0.01% to 0.03% w/v. In another embodiment, the concentration of the flavoring agent is 0.01% w/v, 0.02% w/v, 0.03% w/v, 0.04% w/v, 0.05% w/v.

In another embodiment, the present application provides an esketamine liquid formulation, which further comprises an antioxidant. The antioxidant can effectively prevent or delay the oxidation of the formulation, which may prevent the oxidative deterioration of drugs and their formulations, as well as problems such as discoloration and precipitation caused by oxidation, and thus increase the stability of drugs. In an embodiment, the antioxidant of the liquid formulation is ethylenediaminetetraacetic acid (EDTA, edetic acid) or a sodium or calcium salt thereof, vitamin E, gallate, sodium bisulfite, ascorbic acid or a salt thereof, butylhydroxyanisole, or tocopherol, or a combination thereof.

In an embodiment, the present application provides an esketamine liquid formulation, in which the concentration of the antioxidant is from 0.010% to 0.020% w/v. In another embodiment, the concentration of the antioxidant is from 0.010% to 0.015% w/v. In another embodiment, the concentration of the antioxidant of the liquid formulation is 0.010% w/v, 0.011% w/v, 0.012% w/v, 0.013% w/v, 0.014% w/v, 0.015% w/v, 0.016% w/v, 0.017% w/v, 0.018% w/v, 0.019% w/v, or 0.02% w/v.

In an embodiment, the esketamine liquid formulation further comprises a preservative. In some embodiments, the preservative is selected from one or more of methyl paraben, ethyl paraben, propyl paraben, butyl paraben, methyl paraben sodium, ethyl paraben sodium, propyl paraben sodium, butyl paraben sodium, sorbic acid, potassium sorbate, sodium sorbate, benzoic acid, sodium benzoate, benzyl alcohol, benzalkonium bromide, benzalkonium chloride, trichlorobutanol, resorcinol, and sodium ethylenediaminetetraacetate (sodium edetate).

In one embodiment, the esketamine liquid formulation further comprises an osmotic pressure regulator. In some embodiments, the osmotic pressure regulator is selected from one or more of sodium chloride, potassium nitrate, boric acid, and glucose, preferably sodium chloride.

In one embodiment, the esketamine liquid formulation further comprises a permeation enhancer. In some embodiments, the permeation enhancer is selected from one or more of sodium deoxycholate, Tween 80, hydroxypropyl methyl cellulose, sodium dodecyl sulfate, sodium docusate, polysorbate, tetradecyl maltoside, sodium glycocholate, tauroursodeoxycholic acid (TUDCA), lecithin, hydroxypropyl-β-cyclodextrin, sulfobutyl-β-cyclopaste sodium, or PEG400.

A second aspect of the present application specifically provides an esketamine liquid formulation comprising esketamine hydrochloride, cholic acid or a derivative thereof, a tackifier and water; the liquid formulation has a pH in the range of 4.5 - 5.5, and the content of esketamine in the liquid formulation is 0.7% w/v -8.4% w/v.

In one embodiment, the kinds of the tackifiers and their content with the cholic acid or derivatives thereof are as previously described.

In another embodiment, the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof.

In the esketamine liquid formulation provided in the present application, the simultaneous use of a cholic acid or a derivative thereof and a tackifier can make the API in the formulation more fully released in vitro, better promote the permeability of esketamine through the oral mucosa, and synergistically improve the bioavailability of the esketamine liquid formulation.

In an embodiment of the esketamine liquid formulation described in the present application, the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof. In an embodiment, the tackifier is xanthan gum. In another embodiment, the tackifier is hydroxypropyl methyl cellulose. In another embodiment, the tackifier is sodium carboxymethyl cellulose 4000 and sodium carboxymethyl cellulose 12000. In another embodiment, the tackifier is sodium carboxymethyl cellulose 4000 and hydroxypropyl methyl cellulose.

A third aspect of the present application specifically provides an esketamine liquid formulation, which comprises esketamine hydrochloride, a cholic acid or a derivative thereof, a tackifier, a buffer, and water. The pH of the liquid formulation ranges from 4.5 to 5.5, and the content of esketamine in the liquid formulation is from 0.7% w/v to 8.4% w/v.

In an embodiment, the types of tackifiers and buffers as well as the contents of the tackifiers, the buffers, and the cholic acid or derivative thereof are as previously described.

In an embodiment, the present application provides an esketamine liquid formulation, in which the buffer is citric acid.

A fourth aspect of the present application specifically provides an esketamine liquid formulation, which comprises esketamine hydrochloride, a cholic acid or a derivative thereof, a tackifier, an antioxidant, a flavoring agent, a buffer, and water. The pH of the liquid formulation ranges from 4.5 to 5.5, and the content of esketamine in the liquid formulation is from 0.7% w/v to 8.4% w/v.

In an embodiment, the types of tackifiers, antioxidants, flavoring agents, and buffers as well as the contents of the tackifiers, the antioxidants, the flavoring agents, the buffers, and the cholic acid or derivative thereof are as previously described.

In another embodiment, the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof.

In an embodiment of the esketamine liquid formulation described in the present application, the antioxidant is disodium edetate, the flavoring agent is sodium saccharin or maltitol, and the tackifier is one of sodium carboxymethyl cellulose 4000, sodium carboxymethyl cellulose 8000, sodium carboxymethyl cellulose 12000, xanthan gum, and hydroxypropyl methyl cellulose, or a combination thereof. In an embodiment, the antioxidant is disodium edetate, the flavoring agent is sodium saccharin or maltitol, and the tackifier is xanthan gum. In another embodiment, the antioxidant is disodium edetate, the flavoring agent is sodium saccharin or maltitol, and the tackifier is hydroxypropyl methyl cellulose. In another embodiment, the tackifier is sodium carboxymethyl cellulose 4000 and sodium carboxymethyl cellulose 12000. In another embodiment, the antioxidant is disodium edetate, the flavoring agent is sodium saccharin or maltitol, and the tackifier is sodium carboxymethyl cellulose 4000 and hydroxypropyl methyl cellulose.

In an embodiment, the present application provides an esketamine liquid formulation, in which the buffer is citric acid.

A fifth aspect of the present application provides a use of the esketamine liquid formulation in the manufacture of a medicament for preventing, alleviating or treating depression, in which the medicament is useful for treating major depression, unipolar depression, refractory depression, intractable depression, anxiety depression, or bipolar depression.

In an embodiment, the present application provides a use of an esketamine liquid formulation in the manufacture of a medicament for preventing, alleviating or treating depression, in which the liquid formulation is administered to a buccal membrane of the oral cavity of a patient.

A sixth aspect of the present application provides a method for preventing, alleviating or treating depression in a patient using an esketamine liquid formulation, which comprises administering to the patient a therapeutically effective amount of the esketamine liquid formulation.

In an embodiment, the present application provides a method for preventing, alleviating, or treating depression in a patient using an esketamine liquid formulation, in which the depression is major depression, unipolar depression, refractory depression, intractable depression, anxiety depression, or bipolar depression.

In an embodiment, the present application provides a method for preventing, alleviating or treating depression in a patient using an esketamine liquid formulation, in which the esketamine liquid formulation is administered to a buccal membrane of the oral cavity of a patient.

A seventh aspect of the present application provides an esketamine liquid formulation for use in preventing, alleviating or treating depression in a patient, in which the depression is major depression, unipolar depression, refractory depression, intractable depression, anxiety depression, or bipolar depression.

In an embodiment, an esketamine liquid formulation is used for preventing, alleviating or treating depression in a patient, in which the liquid formulation is administered to the buccal membrane of the oral cavity of a mammal.

In an eighth aspect, the present application provides a method for preparing the aforementioned esketamine liquid formulation, which comprises the following preparation steps:
a) weighing a formulation amount of esketamine hydrochloride, adding purified water, fully stirring to dissolve, then sequentially adding a formulation amount of optional antioxidant such as disodium edetate, optional buffer such as citric acid, and optional flavoring agent such as sodium saccharin, and fully stirring to dissolve;
b) adding the cholic acid or its derivative in the formulation, optionally the tackifier, and fully stirring to dissolve;
c) adjusting the pH to a corresponding value with sodium hydroxide;
d) transferring the solution to a specific volumetric flask, and bringing to volume with water to the graduation line; and
e) passing the solution through a 0.45 µm filter membrane after bringing to volume, and filling the filtrate into a prefilled syringe to obtain the finished product of the formulation.

The beneficial effects of the present application are as follows:
(1) The esketamine liquid formulation provided in the present application is rapidly absorbed, takes effect quickly within 4 to 7 minutes, and its AUC0-240min can be greater than 7000 ng/mL. The esketamine liquid formulation has high bioavailability, has no crystallization phenomenon, and has high stability. The liquid formulation cannot only meet the needs of large-scale processing and production, but also reduce the medication risk of patients.
(2) The esketamine liquid formulation provided by the present application can effectively improve the bioavailability of active substances by using a cholic acid or a derivative thereof. By further adding a tackifier to increase the residence time of the liquid formulation in the oral mucosa, the absorbed amount of esketamine can be further improved, and the bioavailability of the esketamine liquid formulation can be synergistically improved.
(3) The administration mode of the esketamine liquid formulation provided by the present application is simple and easy, and the patient compliance is greatly increased. Due to the small dosage and small volume, the irritation to the mucous membrane can be greatly reduced, and the risk of abuse can be reduced at the same time.

Concentrations of all ingredients are expressed in weight/volume% (% w/v) unless otherwise specified. As is generally understood, the % w/v value refers to the amount of a particular component or ingredient in a preparation. It is well known that equivalent concentrations can be expressed in different units. For example, a concentration of 0.1% w/v can also be expressed as 1 mg/ml of a solution.

### Detailed Description

The present application discloses an esketamine liquid formulation administered through oral mucosa and the use thereof, which can be realized by those skilled in the art in light of the disclosure of the present application and appropriate improvement of the process parameters. In particular, it should be noted that all similar substitutions and modifications will be apparent to those skilled in the art and are deemed to be included in the present application. The methods and applications of the present application have been described with reference to the preferred embodiments, and it will be apparent to those involved that the techniques of the present application will be realized and applied by making modifications or appropriate alterations and combinations of the methods and applications described herein without departing from the content, spirit and scope of the present application. The reagents or instruments used in the present application are commercially available.

Instrument:
S220-K pH meter, SHJ-6AB water-bath kettle with magnetic stirrer, ME204T/02 electronic balance, ME3002T/02 electronic balance, DSC-800T fully-automatic transdermal diffusion instrument, Dionex U300 high performance liquid chromatograph.

### Example 1

### Formulation:

| | |
|---|---|
| Esketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium deoxycholate | 0.05 mg |
| Water | q.s. to 1 ml |

The preparation method comprised the following preparation steps:
a) weighing the formulation amount of esketamine hydrochloride, adding purified water of 80% of the total mass, fully stirring to dissolve, then sequentially adding the formulation amount of disodium edetate, citric acid and sodium saccharin, and fully stirring to dissolve;
b) adding sodium deoxycholate and sodium carboxymethyl cellulose 4000 corresponding to different formulations, and fully stirring to dissolve;
c) adjusting the pH to 4.5-5.5 with sodium hydroxide;
d) transferring the solution to a specific volumetric flask, and bringing to volume with water to the graduation line of 10 ml; and
e) passing the solution through a 0.45 µm filter membrane after bringing to volume, and filling the filtrate into a 1 ml prefilled syringe (purchased from Shandong Zibo Minkang Pharmaceutical Co., Ltd.), to obtain the finished product of the formulation by 1 ml/piece.

### Example 2

### Formulation:

| | |
|---|---|
| Esketamine hydrochloride | 64.6 mg |
| Citric acid | 1.5 mg |
| Disodium edetate | 0.12 mg |
| Sodium saccharin | 0.03 mg |
| Sodium carboxymethylcellulose 8000 | 1 mg |
| Sodium deoxycholate | 0.05 mg |
| Water | q.s. to 1 ml |

Example 1 was referred to for the preparation method.

### Example 3

### Formulation:

| | |
|---|---|
| Esketamine hydrochloride | 64.6 mg |
| Citric acid | 1.5 mg |
| Disodium edetate | 0.12 mg |
| Sodium saccharin | 0.3 mg |
| Sodium carboxymethylcellulose 12000 | 1 mg |
| Sodium deoxycholate | 0.05 mg |
| Water | q.s. to 1 ml |

Example 1 was referred to for the preparation method.

### Example 4

### Formulation:

| | |
|---|---|
| Esketamine hydrochloride | 64.6 mg |
| Citric acid | 1.5 mg |
| Disodium edetate | 0.12 mg |
| Sodium saccharin | 0.3 mg |
| Xanthan gum | 1 mg |
| Sodium deoxycholate | 0.05 mg |
| Water | q.s. to 1 ml |

Example 1 was referred to for the preparation method.

### Example 5

### Formulation:

| | |
|---|---|
| Esketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 0.2 mg |
| Sodium carboxymethylcellulose 12000 | 0.8 mg |
| Sodium deoxycholate | 0.05 mg |
| Water | q.s. to 1 ml |

Example 1 was referred to for the preparation method.

### Example 6

### Formulation

| | |
|---|---|
| Esketamine hydrochloride | 64.6 mg |
| Citric acid | 1.5 mg |
| Disodium edetate | 0.12 mg |
| Sodium saccharin | 0.3 mg |
| hydroxypropyl methyl cellulose | 1 mg |
| Sodium deoxycholate | 0.05 mg |
| Water | q.s. to 1 ml |

Example 1 was referred to for the preparation method.

### Example 7

### Formulation

| | |
|---|---|
| Esketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 0.2 mg |
| hydroxypropyl methyl cellulose | 0.8 mg |
| Sodium deoxycholate | 0.05 mg |
| Water | q.s. to 1 ml |

Example 1 was referred to for the preparation method.

### Example 8

### Formulation

| | |
|---|---|
| Esketamine hydrochloride | 32.3 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium deoxycholate | 0.05 mg |
| Water | q.s. to 1 ml |

Example 1 was referred to for the preparation method.

### Example 9

### Formulation

| | |
|---|---|
| Esketamine hydrochloride | 16.14 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium deoxycholate | 0.05 mg |
| Water | q.s. to 1 ml |

Example 1 was referred to for the preparation method.

### Example 10

### Formulation

| | |
|---|---|
| Esketamine hydrochloride | 64.6 mg |
| Citric acid | 1.5 mg |
| Disodium edetate | 0.12 mg |
| Sodium saccharin | 0.3 mg |
| Sodium carboxymethylcellulose 4000 | 1 mg |
| Sodium glycinodeoxycholate | 0.05 mg |
| Water | q.s. to 1 ml |

### Example 1 was referred to for the preparation method.

### Example 11 Stability test of esketamine hydrochloride buccal liquid of the present application

Esketamine hydrochloride buccal liquid is prone to produce the following two impurities during preparation and storage:

| Name | Structural formula | Molecular formula and molecular weight | Chemical name |
|---|---|---|---|
| Impurity B | 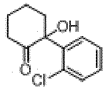 | C₁₂H₁₃O₂ | 2-(2-chlorophenyl)-2-(hydroxy) cyclohexanone |
| Impurity C | 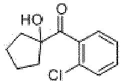 | C₁₂H₁₃ClO₂ | 1-hydroxycyclopentyl-2-chlorophenylmethanone |

The above two impurities are commercially available.

The present inventor(s) performed long-term stability experiments on the samples prepared in Examples 1 to 10 in an environment of 25 °C ± 2 °C and RH 60% ± 5%.
1. Method for detecting the content of ketamine:
   The detection was according to high performance liquid chromatography (general rule 0512).
   Chromatographic conditions: octadecylsilane bonded silica was used as a filler; 0.95 g of sodium hexanesulfonate was dissolved in 1 L of a mixed solution of acetonitrile and water (acetonitrile:water = 25:75), then 4 ml of glacial acetic acid was added and mixed well to form the mobile phase; the detection wavelength was 215 nm; the flow rate was 1.0 ml per minute; the column temperature was 30°C; and the injection volume was 20 µl.
2. Method for detecting ketamine related substances:
   The detection was according to high performance liquid chromatography (general rule 0512).

Chromatographic conditions: octadecylsilane bonded silica was used as a filler (Agela MP C18 4.6 × 150 mm, 5 µm or equivalent column efficiency); 25 mM phosphate buffer solution (3.4 g of potassium dihydrogen phosphate was dissolved in 1000 ml of water, and the pH was adjusted to 2.5 with phosphoric acid) was used as mobile phase A, and acetonitrile was used as mobile phase B. Gradient elution was performed according to the table below, and the detection wavelength was 215 nm; the column temperature was 30°C; the flow rate was 1.0 ml per minute; and the injection volume was 20 µl.

| Time (minutes) | Mobile Phase A (%) | Mobile Phase B (%) |
|---|---|---|
| 0 | 85 | 15 |
| 35 | 60 | 40 |
| 37 | 60 | 40 |
| 65 | 20 | 80 |
| 70 | 20 | 80 |
| 71 | 85 | 15 |
| 75 | 85 | 15 |

**TABLE 4 Results of long-term test of Examples 1-10**

| Formulation | Time (months ) | pH | Content (%) | Related substances (%) | | | |
|---|---|---|---|---|---|---|---|
| | | | | Impurity B | Impurity C | Other individual impurity | Total impurities |
| Example 1 | 0 | 5.1 | 98.48 | Not detected | Not detected | Not detected | Not detected |
| | 3 | 5.3 | 101.67 | Not detected | Not detected | 0.011 | 0.017 |
| | 6 | 5.2 | 102.42 | Not detected | Not detected | 0.022 | 0.026 |
| Example 2 | 0 | 5.1 | 99.43 | Not detected | Not detected | Not detected | Not detected |
| | 3 | 5.1 | 101.61 | Not detected | Not detected | 0.023 | 0.029 |
| | 6 | 5.2 | 102.15 | Not detected | Not detected | 0.036 | 0.042 |
| Example 3 | 0 | 5.0 | 99.85 | Not detected | Not detected | Not detected | Not detected |
| | 3 | 5.1 | 100.33 | Not detected | Not detected | 0.031 | 0.039 |
| | 6 | 5.2 | 100.75 | Not detected | Not detected | 0.046 | 0.052 |
| Example 4 | 0 | 5.0 | 100.12 | Not detected | Not detected | Not detected | Not detected |
| | 3 | 5.0 | 100.73 | Not detected | Not detected | 0.017 | 0.021 |
| | 6 | 5.1 | 101.22 | 0.01 | 0.01 | 0.024 | 0.046 |
| Example 5 | 0 | 4.9 | 99.27 | Not detected | Not detected | Not detected | Not detected |
| | 3 | 5.1 | 101.53 | Not detected | Not detected | 0.018 | 0.023 |
| | 6 | 5.2 | 102.26 | Not detected | 0.01 | 0.026 | 0.042 |
| Example 6 | 0 | 5.0 | 102.33 | Not detected | Not detected | Not detected | Not detected |
| | 3 | 5.1 | 100.47 | Not detected | Not detected | 0.033 | 0.038 |
| | 6 | 5.2 | 101.29 | Not detected | Not detected | 0.047 | 0.055 |
| Example 7 | 0 | 5.1 | 101.74 | Not detected | Not detected | Not detected | Not detected |
| | 3 | 5.3 | 101.05 | Not detected | Not detected | 0.018 | 0.024 |
| | 6 | 5.3 | 100.61 | Not detected | 0.01 | 0.031 | 0.049 |
| Example 8 | 0 | 5.0 | 99.77 | Not detected | Not detected | Not detected | Not detected |
| | 3 | 5.3 | 100.67 | Not detected | Not detected | 0.014 | 0.018 |
| | 6 | 5.2 | 101.42 | Not detected | Not detected | 0.021 | 0.029 |
| Example 9 | 0 | 5.1 | 99.56 | Not detected | Not detected | Not detected | Not detected |
| | 3 | 5.2 | 100.25 | Not detected | Not detected | 0.018 | 0.024 |
| | 6 | 5.3 | 100.91 | 0.01 | Not detected | 0.025 | 0.042 |
| Example 10 | 0 | 5.0 | 100.17 | Not detected | Not detected | Not detected | Not detected |
| | 3 | 5.1 | 100.82 | Not | Not | 0.011 | 0.015 |
| | | | | detected | detected | | |
| | 6 | 5.1 | 101.61 | Not detected | 0.01 | 0.023 | 0.039 |

According to the results of the long-term test in Table 4, it can be seen that when the pharmaceutical formulations obtained in the present application was placed at room temperature for up to 6 months, the total impurities of the esketamine hydrochloride buccal liquid were not more than 0.5%, and the maximum individual impurities were not more than 0.2%. Therefore, the quality of the pharmaceutical formulations still met the quality standard, and the quality was stable.

### Example 12 In vitro penetration test of esketamine hydrochloride buccal liquid of the present application

In this study, the Franz diffusion cell method was used, the phospholipid biomimetic barrier PermeaPad^{®} (biomimetic membrane) was selected to simulate the oral mucosa, and the diffusion medium was PBS buffer. After the diffusion cell reached 37°C, firstly add 0.5 ml of artificial saliva above the biomimetic membrane, and then add 0.2 ml of formulation solutions. The sampling time points are 10 min, 20 min, 30 min, 45 min, 60 min, 180 min, 240 min, 300 min, and 360 min. Using the Waste mode, 1 ml sample was took at each time point, and the content in liquid phase was detected.

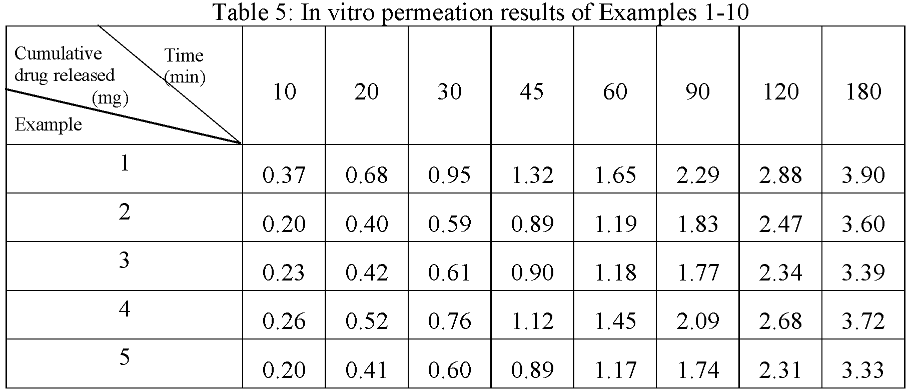

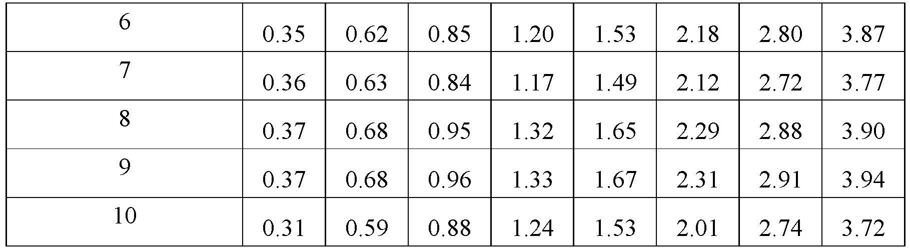

### Comparative Example 1 Esketamine Hydrochloride Nasal Spray (Prepared with reference to Patent CN 111297803 A)

### Components of the formulation

| | |
|---|---|
| Esketamine hydrochloride | 161.4 mg |
| Citric acid | 1.5 mg |
| Disodium edetate | 0.12 mg |
| Sodium hydroxide | adjusted pH to 4.52 |
| Water | q.s. to 1 ml |

### Product specifications: Each spray contains 7 mg of esketamine

### Product specifications: 7 mg of esketamine in each spray

The preparation method comprised the following preparation steps:
a) weighing the formulation amount of D-ketamine hydrochloride, adding a certain amount of purified water, fully stirring to dissolve, then sequentially adding the formulation amount of disodium edetate, and citric acid, and fully stirring to dissolve;
b) adjusting the pH to 4.5 with sodium hydroxide;
c) transferring the solution to a specific volumetric flask, and bringing to volume with water to the graduation line; and
d) passing the solution through a 0.45 µm filter membrane after bringing to volume, and filling the filtrate as needed.

### Comparative Example 2. Esketamine hydrochloride oral dissolving film 1 (Prepared with reference to Patent CN 111447920 A)

### Components in the formulation

| | |
|---|---|
| Esketamine hydrochloride | 4.3232 g |
| Hydroxypropyl methylcellulose | 9.00 g |
| Hydroxypropylcellulose | 3.00 g |
| PEG400 | 300 g |
| Carbomer 974P | 0.01 g |
| Hydroxypropyl methyl-β-cyclodextrin | 0.37 g |

The preparation method comprised the following preparation steps:
a) weighing the formulation amount of esketamine hydrochloride, adding a certain amount of purified water, fully stirring to dissolve, then sequentially adding the formulation amount of hydroxypropyl methyl cellulose, hydroxypropyl cellulose and PEG400 for fully stirring to dissolve;
b) adding carbomer 974P and Hydroxypropyl methyl-β-cyclodextrin corresponding to different formulations, and fully stirring to dissolve; and
c) after standing for defoaming, coating, oven drying, and knocking into required specifications with a die.

### Comparative Example 3. Esketamine hydrochloride oral dissolving film 2 (Prepared with reference to Patent CN 111447920 A)

### Components in the formulation

| | |
|---|---|
| Esketamine hydrochloride | 4.3232 g |
| Hydroxypropyl methylcellulose | 9.00 g |
| Hydroxypropylcellulose | 3.00 g |
| PEG400 | 300 g |
| Sodium deoxycholate | 0.003 g |

The preparation method was referred to Comparative Example 2, using sodium deoxycholate instead of carbomer 974P and hydroxypropyl methyl-β-cyclodextrin.

### Test Example 1 In vivo pharmacokinetic experiments

### 1. Experimental grouping

**Table 6: Grouping of animals**

| Serial number | Administration route |
|---|---|
| Comparative Example 1 | Unilateral nasal administration |
| Example 1 | Unilateral buccal membrane administration |
| Comparative Example 2 | Unilateral buccal membrane administration |
| Comparative Example 3 | Unilateral buccal membrane administration |
| Comparative Example 2 | Sublingual administration |
| Comparative Example 3 | Sublingual administration |

### 2. Experimental protocol

In this experiment, New Zealand white rabbits, 2.5 kg ~ 3.0 kg, were used, 6 rabbits in each group, and half male and half female. In Comparative Example 1, unilateral nasal administration was used, and the dosing amount was 0.05 ml/animal (1 spray). In Example 1, unilateral buccal liquid administration was used, and the dosing amount was 0.125 ml/animal. In Comparative Example 2 and Comparative Example 3,unilateral buccal membrane administration was used, and the dosing amount was 1 tablet/animal (7 mg). In Comparative Example 2 and Comparative Example 3, sublingual administration was used, and the dosing amount was 1 tablet/animal (7 mg). Before blood collection, the rabbits were placed in a fixed box for rabbits. The blood collection method was ear vein blood collection. The time points for blood collection were: 0 min (before administration), 2 min, 5 min, 10 min, 15 min, 30 min, 60 min, 120 min and 240 min. At each time point, about 1 mL of whole blood was transferred into a blood collection tube containing sodium heparin, and centrifuged at 4000 rpm for 10 min to obtain plasma samples. The supernatant was subpackaged in 3 portions into 0.5 ml EP tubes (the first 2 portions were filled with 150 µl, and the excess plasma was filled in the third cryopreservation tube as a backup sample). The plasma samples were stored at ≤-80°C after the subpackaging.

### 3. Experimental results

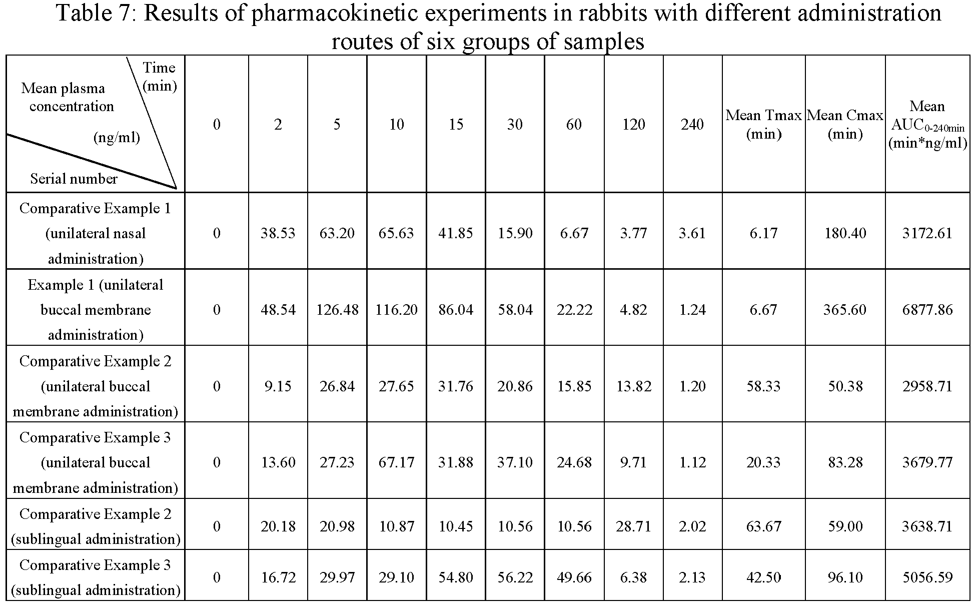

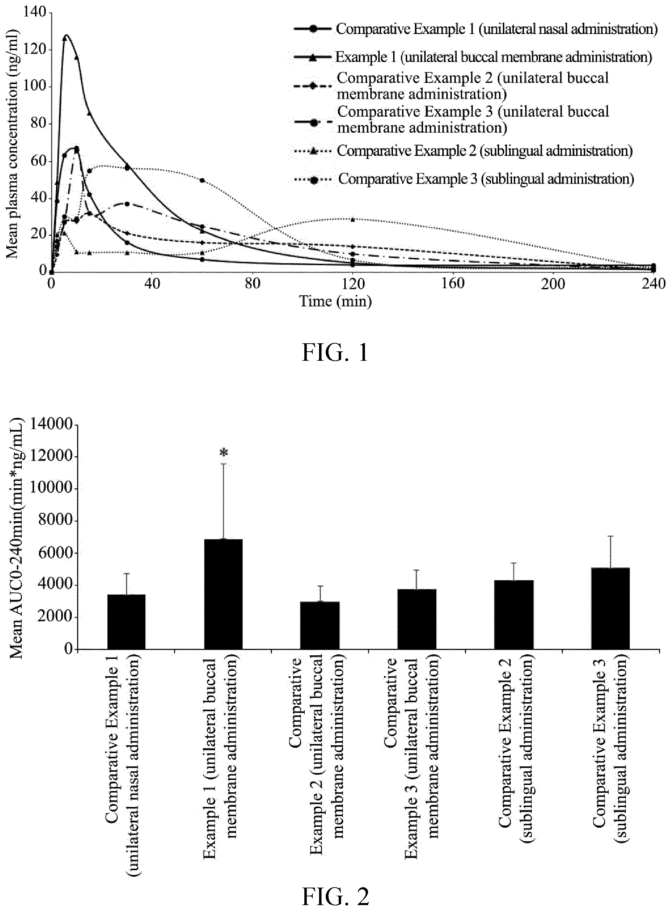

As can be seen from Table 7 and FIG. 1, in various administration route groups of esketamine hydrochloride for rabbits, the Tmax in the buccal administration group of buccal liquid (Example 1) was similar to that in the nasal administration group (Comparative Example 1), which was in the range of 4 minutes to 7 minutes, while the Tmax in the oral dissolving film group (Comparative Example 2 and Comparative Example 3) was in the range of 20 minutes to 60 minutes, indicating that the onset time of action of the buccal membrane administration of the esketamine hydrochloride buccal liquid in the present application was similar to that of nasal administration of the nasal spray, while the onset time of action of the oral dissolving film was slow.

As can be seen from the data of average Cmax, compared with the nasal administration group (Comparative Example 1), the Cmax in the buccal administration group of buccal liquid (Example 1) was significantly increased, which was increased by about 1 time; while the Cmax in the oral dissolving film group (Comparative Example 2 and Comparative Example 3) was lower than that in the nasal administration group, which decreased by about 1 to 2 times, indicating that the drug absorption in the buccal membrane administration of esketamine hydrochloride buccal liquid in the present application was higher than that in nasal administration.

As can be seen from Table 7 and FIG. 2, compared with the nasal administration group (Comparative Example 1), the AUC0-240min in the buccal administration group of buccal liquid (Example 1) was significantly increased (p < 0.05), which was increased by about 1 time; while the AUC0-240min in the oral dissolving film group (Comparative Example 2 and Comparative Example 3) was not significantly different from that in the nasal administration group (p > 0.05). The results were consistent with the results of Cmax, further demonstrating that the in vivo drug absorption in the buccal membrane administration of esketamine hydrochloride buccal liquid in the present application was higher than that in nasal administration and oral dissolving film administration.

To sum up, the in vivo drug absorption in the buccal membrane administration of esketamine hydrochloride buccal liquid is significantly higher than that in nasal administration and oral dissolving membrane administration, which is increased by about 1 time, and the onset time of action of these two routes is similar, about 4 minutes to 7 minutes, with a faster onset speed of action than that in oral dissolving membrane administration.

The technical solution of the present application can also be used in a buccal liquid buccal membrane administration system of R-ketamine, ketamine or a pharmaceutically acceptable salt, and the object of the present invention can be achieved by adjusting or changing the content of active ingredients, types and dosages of auxiliary materials that can be used in pharmacy, and the like.

Although the embodiments disclosed in the present application are as above, the contents described are only the embodiments adopted for the convenience of understanding the present application, and are not used to limit the present application. Any person skilled in the art to which this application belongs may make any modifications and changes in the form and details of implementation without departing from the idea and scope disclosed in this application, but the scope of protection of this application shall still be subject to the scope defined in the appended claims.

## Claims

1. An esketamine liquid formulation for oral mucosa administration, comprising esketamine or a pharmaceutically acceptable salt thereof with a concentration of 0.5% w/v - 8.5% w/v, cholic acid or a derivative thereof, and water; wherein the liquid formulation has a pH in the range of 2.5 - 5.7.

2. The esketamine liquid formulation according to claim 1, wherein the liquid formulation comprises esketamine or a pharmaceutically acceptable salt thereof with a concentration of 0.5% w/v - 8.5% w/v, cholic acid or a derivative thereof with a concentration of 0.001% w/v - 0.015% w/v, and water; the liquid formulation has a pH in the range of 2.5 - 5.7.

3. The esketamine liquid formulation according to claim 2, wherein the concentration of esketamine in the liquid formulation is 0.7% w/v - 8.4% w/v; preferably 1.4% w/v - 6.3% w/v; more preferably 1.4% w/v - 5.6% w/v.

4. The esketamine liquid formulation according to claim 2, wherein the cholic acid or a derivative thereof in the liquid formulation is selected from one of sodium taurate, sodium taurodeoxycholate, sodium deoxycholate, sodium cholate, sodium glycinodeoxycholate, or a combination thereof, preferably sodium deoxycholate or sodium taurate.

5. The esketamine liquid formulation according to claim 2, wherein the concentration of cholic acid or derivative thereof in the liquid formulation is 0.001% w/v - 0.012% w/v, preferably 0.001% w/v - 0.010% w/v; more preferably 0.003% w/v - 0.010 w/v.

6. The esketamine liquid formulation according to claim 2, wherein the liquid formulation has a pH in the range of 3.0 - 5.7; preferably 4.0-5.7; more preferably 4.5 - 5.5; particularly preferably 5.0 - 5.5.

7. The esketamine liquid formulation according to any one of claims 1 to 6, wherein the liquid formulation further comprises a tackifier.

8. The esketamine liquid formulation according to claim 7, wherein the tackifier is one of xanthan gum, sodium carboxymethyl cellulose, hydroxypropyl methyl cellulose, methyl cellulose, ethyl cellulose, hydroxyethyl cellulose, polyvinyl alcohol, carbomer, and polyvinylpyrrolidone, or a combination thereof.

9. The esketamine liquid formulation according to claim 8, wherein the sodium carboxymethyl cellulose is sodium carboxymethyl cellulose 800, sodium carboxymethyl cellulose 4000, sodium carboxymethyl cellulose 8000, or sodium carboxymethyl cellulose 12000, or a combination thereof.

10. The esketamine liquid formulation according to claim 7, wherein the tackifier has a concentration of 0.01% w/v to 3.5% w/v.

11. The esketamine liquid formulation according to any one of claims 1 to 10, wherein the liquid formulation further comprises one or more of the following auxiliary materials: a buffer, a flavoring agent, an antioxidant, an osmotic pressure regulator, and a preservative; alternatively, the liquid formulation further comprises one or more of the following auxiliary materials: a buffer, a flavoring agent, an antioxidant, an osmotic pressure regulator, a preservative, and a permeation enhancer.

12. The esketamine liquid formulation according to claim 11, wherein the liquid formulation further comprises a buffer, a flavoring agent, and an antioxidant.

13. The esketamine liquid formulation according to claim 11 or 12, wherein the buffer is one of citric acid, sodium citrate, acetic acid, sodium acetate, lactic acid, sodium dihydrogen phosphate, disodium hydrogen phosphate, succinic acid, boric acid, sodium borate, tartaric acid, and fumaric acid, or a combination thereof.

14. The esketamine liquid formulation according to claim 11 or 12, wherein the flavoring agent is one of sodium saccharin, fructose, sucralose, steviosin, menthol, maltitol, xylitol, aspartame, sodium cyclamate, saccharin, neohesperidin, thaumatin, stevia, and acesulfame, or a combination thereof.

15. The esketamine liquid formulation according to claim 11 or 12, wherein the antioxidant is one of disodium edetate, vitamin E, gallate, sodium bisulfite, ascorbic acid or a salt thereof, butylhydroxyanisole, and tocopherol, or a combination thereof.

16. Use of the esketamine liquid formulation according to any one of claims 1 to 15 in the manufacture of a medicament for preventing, alleviating or treating depression.

17. The use according to claim 16, wherein the depression is major depression, unipolar depression, refractory depression, intractable depression, anxiety depression, or bipolar depression.

18. The use according to claim 16 or 17, wherein the liquid formulation is administered to a buccal membrane of the oral cavity of a patient.

19. A method for preventing, alleviating or treating depression in a patient using the esketamine liquid formulation according to any one of claims 1 to 15, comprising administering a therapeutically effective amount of the esketamine liquid formulation to a buccal membrane of the oral cavity of the patient.

20. The method according to claim 19, wherein the depression is major depression, unipolar depression, refractory depression, intractable depression, anxiety depression, or bipolar depression.

21. The esketamine liquid formulation according to any one of claims 1 to 15, for use in preventing, alleviating or treating depression in a patient.

22. The esketamine liquid formulation according to claim 21, for use in preventing, alleviating or treating depression in a patient, wherein the depression is major depression, unipolar depression, refractory depression, intractable depression, anxiety depression, or bipolar depression.

23. The esketamine liquid formulation according to claim 21 or 22, for use in preventing, alleviating or treating depression in a patient, wherein the liquid formulation is administered to a buccal membrane of the oral cavity of the patient.
